(19) Europäisches Patentamt

European Patent Office

Office européen des brevets

(11) **EP 1 346 712 B1**

(12) **EUROPEAN PATENT SPECIFICATION**

(45) Date of publication and mention
of the grant of the patent:
**18.01.2006 Bulletin 2006/03**

(51) Int Cl.:
***A61F 13/15*** *(2006.01)*

(21) Application number: **03002846.8**

(22) Date of filing: **07.02.2003**

(54) **Absorbent article**

Absorbierender Artikel

Article absorbant

(84) Designated Contracting States:
**DE GB SE**

(30) Priority: **22.03.2002 JP 2002082179**

(43) Date of publication of application:
**24.09.2003 Bulletin 2003/39**

(73) Proprietor: **KAO CORPORATION**
**Chuo-ku,**
**Tokyo (JP)**

(72) Inventors:
• **Toyoshima, Yasuo**
**Ichikai-machi,**
**Haga-gun,**
**Tochigi (JP)**
• **Sakamoto, Noriko**
**Ichikai-machi,**
**Haga-gun,**
**Tochigi (JP)**
• **Sugiura, Hiroko**
**Ichikai-machi,**
**Haga-gun,**
**Tochigi (JP)**
• **Miyamoto, Takanobu**
**Ichikai-machi,**
**Haga-gun,**
**Tochigi (JP)**

(74) Representative: **Vossius & Partner**
**Siebertstrasse 4**
**81675 München (DE)**

(56) References cited:
**US-A- 5 817 394**

• **PATENT ABSTRACTS OF JAPAN vol. 1997, no. 10, 31 October 1997 (1997-10-31) & JP 09 168562 A (KAO CORP), 30 June 1997 (1997-06-30)**
• **PATENT ABSTRACTS OF JAPAN vol. 1997, no. 05, 30 May 1997 (1997-05-30) & JP 09 003755 A (DAIWABO CO LTD), 7 January 1997 (1997-01-07)**
• **PATENT ABSTRACTS OF JAPAN vol. 1997, no. 10, 31 October 1997 (1997-10-31) & JP 09 158022 A (DAIWABO CO LTD), 17 June 1997 (1997-06-17)**
• **PATENT ABSTRACTS OF JAPAN vol. 1997, no. 08, 29 August 1997 (1997-08-29) & JP 09 111631 A (DAIWABO CO LTD), 28 April 1997 (1997-04-28)**
• **PATENT ABSTRACTS OF JAPAN vol. 1998, no. 03, 27 February 1998 (1998-02-27) & JP 09 296325 A (TOYOBO CO LTD), 18 November 1997 (1997-11-18)**

**Description**

The present invention relates to an absorbent article the topsheet of which is less prone to cling to the skin and which hardly bunches up and exhibits excellent absorbing performance.

The performance requirements that absorbent articles such as a sanitary napkin and a disposable diaper are required to satisfy include (1) absorptivity and retentivity for waste from a wearer, such as menstrual blood or urine, for preventing leakage or failure and (2) surface characteristics not to give discomfort to a wearer and not to cause skin rashes due to overhydration or irritation.

To meet these requirements, it is known to use a bulky sheet with raised portions on the wearer's side as a topsheet of the absorbent articles. However, conventional absorbent articles using such a bulky sheet have such problems that the topsheet is apt to cling to the wearer's body to give the wearer discomfort or a gap tends to be formed between the topsheet and the underlying absorbent member. The gap hinders smooth pass of liquid from the topsheet to the absorbent member, and the liquid remaining in the topsheet can cause overhydration or a rash, and its outstanding color is likely to give a dirty impression to a user.

JP-A-9-168562 proposes fixing an extensible topsheet in its extended state. The purpose of extending the topsheet is to curve the absorbent article. The publication is completely silent on other purposes of extending the topsheet than curving.

JP-A-9-3755 discloses nonwoven fabric with a textured surface designed to serve as a female member of a mechanical fastener in disposable diapers, etc., which is produced by superposing a layer containing heat shrinkable fiber and a layer containing heat non-shrinkable fiber, and heat embossing the two layers thereby bonding the two layers in parts and simultaneously causing the layer containing the heat shrinkable fiber to shrink. The disclosed nonwoven fabric does not exhibit elastomeric behavior. As is natural, there is no mention as to fixing the nonwoven fabric in its extended state.

An object of the present invention is to provide an absorbent article of which the topsheet hardly clings to the skin while worn and is capable of smoothly passing liquid to an absorbent member thereby preventing overhydration or a rash and giving a clean impression and which hardly bunches up.

The present invention accomplishes the above object by providing an absorbent article having a substantially oblong shape and comprising a liquid permeable topsheet, a liquid impermeable backsheet, and a liquid retentive absorbent member interposed between the topsheet and the backsheet, wherein the topsheet is a bulky sheet which exhibits elastomeric behavior as a whole and comprises a first layer disposed on the side of a wearer and a second layer disposed on the side of the absorbent member, the first layer and the second layer being partially bonded together at joints in a prescribed pattern, the first layer having protrusions on the side of a wearer in portions other than the joints, the second layer being made of a material exhibiting elastomeric behavior, and the topsheet is fixed in an extended state in at least one of the longitudinal direction and the width direction of the absorbent article.

The present invention will be more particularly described with reference to the accompanying drawings, in which:

Fig. 1 is a perspective of a preferred example of the bulky sheet used in the present invention;
Fig. 2 is an exaggerated cross-sectional view of the bulky sheet of Fig. 1, taken along line X-X;
Fig. 3 shows a preferred pattern of joints;
Fig. 4(a), Fig. 4(b) and Fig. 4(c) show other patterns of joints;
Fig. 5(a) and Fig. 5(b) show an embodiment of the absorbent article according to the present invention, in which Fig. 5(a) is a perspective with a part cut away, and Fig. 5(b) is a schematic cross-section of Fig. 5(a), taken along line Y-Y;
Fig. 6(a) and Fig. 6(b) show another embodiment of the absorbent article according to the present invention, in which Fig. 6(a) is a plan view, and Fig. 6(b) is a schematic cross-section of Fig. 6(a), taken along line Z-Z;
Fig. 7(a) illustrates a bulky sheet being slitted, and Fig. 7(b) shows the slit of Fig. 7(a) being expanded to make an opening;
Fig. 8(a) and Fig. 8(b) schematically show the absorbent article according to the present invention, in which left Figs. each correspond to Fig. 7(a) and right Figs. each correspond to Fig. 7(b); and
Fig. 9(a) and Fig. 9(b) illustrate still another embodiment of the absorbent article according to the present invention, in which Fig. 9(a) is a perspective view, and Fig. 9(b) is a schematic cross-section of Fig. 9(a), taken along line I-I.

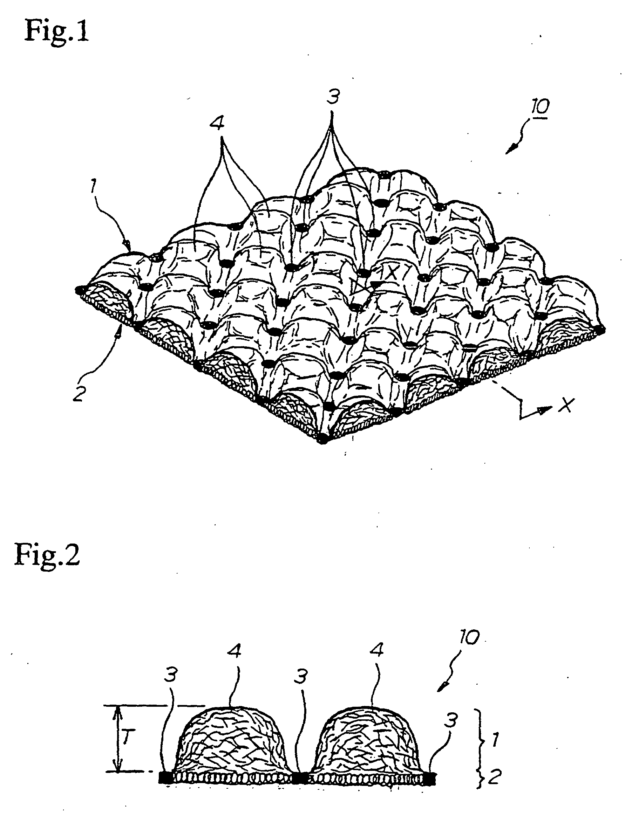

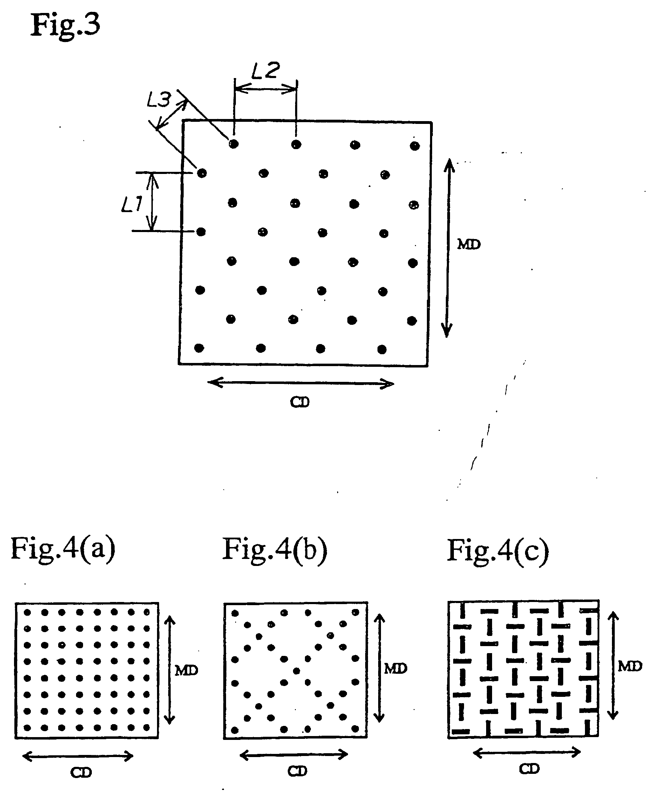

The present invention will be described chiefly based on its preferred embodiments with reference to the accompanying drawings.
A preferred example of the bulky sheet which can be used as a topsheet in the present invention is described by referring to Figs. 1 and 2. The bulky sheet 10 shown in Figs. 1 and 2 consists of a first layer 1 disposed on the wearer's side and a second layer 2 disposed on the side of the absorbent member of an absorbent article. The first layer 1 and the second layer 2 are each made of a fiber aggregate and are superposed on each other and partially joined together at joints 3 in a prescribed pattern. In this embodiment, the joints 3, each having a circular shape in their plan view, are discretely arranged in a lattice pattern as illustrated in Fig. 3. The joints 3 are densified to be thinner and denser than the other portions of the bulky sheet 10.

The joints 3 can be formed by various joining means, for example, heat embossing, ultrasonic embossing or bonding with an adhesive. The individual joints 3 may have an arbitrary shape, such as a circular shape as adapted in the embodiment shown in Figs. 1 through 3, an elliptic shape, a triangular shape, a rectangular shape or a combination thereof The joints 3 may be continuously formed to make a line pattern, such as a pattern of straight lines, curved lines, and intersecting straight lines. Examples of conceivable joints patterns other than the pattern of Fig. 3 are shown in Figs. 4(a)-4(c).

The first layer 1 is made of a fiber aggregate. The portions of the first layer 1 other than the joints 3 with the second layer 2 are raised toward the wearer's side. That is, every portion between adjacent joints 3 (more specifically every rectangular portion having the joints 3 at its four corners when viewed from the above) has a dome-shaped protrusion 4. As a whole, the bulky sheet 10 has a large number of protrusions 4 on its side adapted to face a wearer. Each protrusion 4 is filled with the fiber constituting the first layer 1. In the portions where the first layer 1 forms protrusions, the first layer 1 and the second layer 2 are not bonded together but are in close contact with each other over the entire area. The shape of the protrusions 4 depends chiefly on the form of the fiber aggregate used as a first layer-forming material and the pattern of the joints 3. The first layer 1 is made of an aggregate of fiber different from the fiber making up the second layer 2 in kind and/or composition.

In this embodiment, the second layer 2 is substantially flat between adjacent joints 3 as illustrated in Fig. 2. When viewed as a whole, the bulky sheet 10 is flat on its second layer 2 side and has a large number of protrusions on its first layer 1 side. While the protrusions 4 of the present embodiment are filled with fiber, they may have a hollow structure. In such cases, the first layer-forming material, i.e., a fiber aggregate making the first layer 1, is nonwoven fabric or knitted fabric.

The ratio of the total area of the joints 3 to the area of the bulky sheet 10 which is measured before shrinking a second layer-forming material is preferably 2 to 15%, still preferably 5 to 10%, in order to assure bonding between the two layers 1 and 2 while allowing the first layer-forming material to rise to form the protrusions 4 with sufficient height for bulkiness.

The height T (see Fig. 2) of the protrusions 4 is preferably 0.5 to 5 mm, still preferably 0.5 to 3 mm, to make the bulky sheet 10 bulky and sufficiently deformable in compression. With a height T of 0.5 mm or larger, the contact area with the skin is reduced to prevent overhydration or a rash which might be caused by intimate contact of the skin with the topsheet. With a height T of 5 mm or smaller, the distance the absorbed liquid must travel to reach the second layer 2 is short enough to make the liquid be absorbed by the absorbent member smoothly even under low body pressure.

The height T of the protrusions 4 can be measured as follows. A 30-mm square cut out of a bulky sheet is cut along a line substantially parallel with the longitudinal direction, namely, the fiber orientation direction (the MD) of the nonwoven fabric (fiber aggregate) making the first fiber layer, and passing through the joints 3. A magnified photograph is taken of the cut area under a microscope SZH10 supplied by Olympus Optical Co., Ltd. The real height of from the bottom of a depression (the upper surface of a joint 3) to the apex of a protrusion, calculated from the magnification, is taken as height T.

The bulky sheet 10 preferably has a basis weight of 20 to 150 g/m$^2$, particularly 20 to 80 g/m$^2$, for assuring smooth migration of liquid to the absorbent member while retaining softness as a whole. The basis weight of the bulky sheet is obtained by weighing a specimen of at least 50 mm by 50 mm cut out of a sample with an electronic balance (minimum reading: I mg) and calculating a weight per unit area (m$^2$).

The first layer-forming material which becomes the first layer 1 of the bulky sheet 10 preferably has a basis weight of 5 to 40 g/m$^2$, particularly 5 to 30 g/m$^2$, so that the topsheet may be flexibly deformed in conformity with the wearer's body to give wearing comfort. The second layer-forming material which becomes the second layer 2 preferably has a basis weight of 5 to 50 g/m$^2$, particularly 5 to 40 g/m$^2$, for assuring sufficient shrinkability of a shrinkable fiber, one of the fibers that constitute the second layer, so that the shrinkable fiber may shrink sufficiently to develop extensibility and contractibility. The basis weights of the first and second layer-forming materials refer to those measured before joining them.

The second layer 2 of the bulky sheet 10 is made of a material exhibiting elastomeric behavior. When extended in a planar direction (a direction parallel to the surface of the bulky sheet 10), the second layer 2 develops a prescribed shrinkage stress. Further, the bulky sheet 10 exhibits elastomeric behavior as a whole and shows extensibility and contractibility. Where the bulky sheet 10 is used in its extended state as a topsheet of an absorbent article, it provides an absorbent article which is less prone to cling to the skin and to bunch up and exhibits enhanced absorbing performance while enjoying the advantages of bulkiness, compressive deformability, breathability, and absorptivity of the bulky sheet. As long as the first layer 1 is extensible, it does not matter whether it shows elastomeric behavior. Since both the first and second layers are made of fiber aggregates, the bulky sheet 10 has breathability as a whole.

It is preferred for the bulky sheet 10 to have a recovery from 50% extension of 50% or more, particularly 60 to 90%, for developing sufficient elastomeric behavior. The recovery from extension sometimes varies according to the direction of measurement. Sufficient elastomeric behavior will be exhibited as long as the recovery from extension falls within the above range in at least one of the machine direction (MD) and the cross direction (CD) of the bulky sheet 10.

The recovery from extension is measured as follows. Measurement is made with a tensile/compression tester RTM-100 supplied by Toyo Baldwin in the tensile mode. A 50 mm by 50 mm specimen cut out of a bulky sheet is set between air chucks of the tester at an initial chuck distance of 30 mm. The chuck attached to the load cell (rated output: 5 kg) is moved upward at a pulling speed of 100 mm/min. When the specimen is 50% extended (by 15 mm), the chuck is moved downward to the original position at a speed of 100 mm/min. The readings of the load cell and the extension of the specimen are plotted. The extension recovery is obtained from the plots according to equation:

$$\text{Extension recovery (\%)} = \text{distance of recovery (mm)/maximum extension (=15 mm)} \times 100$$

wherein "distance of recovery" is the distance the chuck travels during unloading until the load cell reading reaches zero.

The first layer is preferably made up of heat bondable fiber, particularly fiber comprising a thermoplastic polymer. Useful thermoplastic polymers include polyolefins, such as polyethylene and polypropylene; polyesters, such as polyethylene terephthalate; and polyamides. Conjugate fibers composed of these thermoplastic polymers, such as core-sheath conjugate fiber and side-by-side conjugate fiber, are also useful. The fiber constituting the first layer 1 is selected from fibers which have substantially no heat shrinkability or fibers which do not shrink at the shrinkage starting temperature of the fiber constituting the second layer 2. The fineness of such non-shrinkable fiber is preferably 0.5 to 20 dtex, still preferably 1.0 to 10 dtex, while dependent on the use of the bulky sheet 10, from the viewpoint of ease of fiber formation and for assuring a satisfactory texture as a topsheet of an absorbent article.

The fiber making up the second layer is a heat shrinkable fiber comprising a thermoplastic polymer. The fiber to be used exhibits elastomeric behavior. Such fibers include self-crimping fiber. Self-crimping fiber can be handled similarly to ordinary fiber for nonwovens before heat application and, when heated at a given temperature, crimps itself in a helical form. Self-crimping fiber having such characteristics exhibits both heat shrinkability and elastomeric behavior.

Self-crimping fibers include conjugate fibers containing two thermoplastic polymers having different shrinkage characteristics in an eccentric core-sheath configuration or a side-by-side configuration. Examples of such self-crimping conjugate fibers are given in JP-A-9-296325 and Japanese Patent 2759331.

The first and second layers may contain fibers other than the above-mentioned fibers, for example, water-absorbing fibers, such as rayon, cotton, and hydrophilic acrylic fiber.

Forms of the fiber aggregate as a first layer-forming material include carded webs, thermal bond nonwovens, water needle nonwovens, needle punch nonwovens, solvent bond nonwovens, spunbonded nonwovens, melt-blown nonwovens, and knitted fabric. Where a carded web is used as a first layer-forming material, it rises to form bulky protrusions filled with the fiber constituting the web, and the fibers are oriented along the contour of the protrusions. In using nonwoven or knitted fabric as a first layer-forming material, it rises to form hollow protrusions. In particular, a carded web becomes a first layer having a structure with very sparse fibers, and the resulting bulky sheet 10 allows viscous liquid to pass through and be retained and exhibits high through-thickness deformabililty when compressed in the thickness direction. The viscous liquid includes soft stool, menstrual blood, cleaning agents or moisture-retaining agents for human bodies, and cleaning agents for inanimate objects.

A carded web is a fiber aggregate before being formed into nonwoven fabric, i.e., a very loose aggregate of fibers that has not been subjected to a post treatment for making nonwovens, such as heat fusion treatment by an air-through process or a calendering process. Where a carded web is used as a first layer-forming material, the fibers of the carded web are bonded to each other by fusion bonding or with a solvent or mechanically entangled with each other either simultaneously with or after joining with a second layer-forming material.

The fiber aggregate as the second layer-forming material includes (1) a carded web containing self-crimping fiber, (2) heat shrinkable nonwoven fabric, such as thermal bond nonwoven, water needle nonwoven, needle punch nonwoven, solvent bond nonwoven, spunbonded nonwoven, and melt-blown nonwoven, and (3) heat shrinkable net. The term "heat shrinkable nonwoven fabric" as used herein means nonwoven fabric which shrinks on heating at a prescribed temperature.

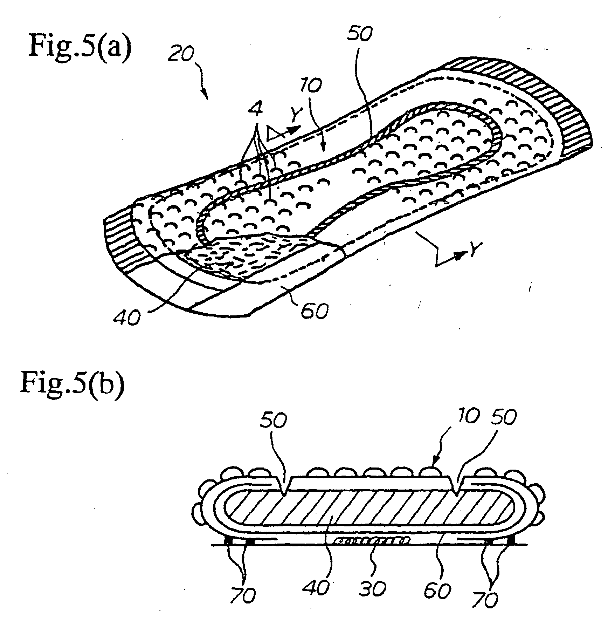

An embodiment of the absorbent article using the above-described bulky sheet as a topsheet will then be described. The sanitary napkin 20 shown in Fig. 5(a) and Fig. 5(b) has a substantially oblong shape and comprises a liquid permeable topsheet 10, a liquid impermeable backsheet 30, and a liquid retentive absorbent member 40 interposed between the topsheet 10 and the backsheet 30. The topsheet used in the sanitary napkin 20 is made of a bulky sheet 10 having the aforementioned structure. The topsheet made of the bulky sheet 10 is fixed in an extended state in the longitudinal direction and/or the width direction of the sanitary napkin (absorbent article). More specifically, the bulky sheet 10 is put on the upper side (the side to face with a wearer) of the absorbent member 40 as extended in the longitudinal direction and/or the width direction of the sanitary napkin and fixed to the upper side of the absorbent member 40 by heat embossing, ultrasonic embossing or with an adhesive, such as a hot-melt adhesive, while maintained in its extended state. The bulky sheet 10 in the napkin 20 shown in Fig. 5(a) and Fig. 5(b) is wrapped around both side edges of the absorbent member 40 and bonded to the backsheet 30 disposed under the absorbent member 40. The portion of the bulky sheet 10 outside the embossed parts (the parts having a leak preventive groove 50 formed by the embossing) may be in either an extended state or a non-extended state. Numeral 60 in Fig. 5(b) indicates a water-repellent leakproof sheet, which is disposed to ensure side leak prevention.

Having the bulky sheet 10 fixed in a longitudinally and/or widthwise extended state as a topsheet, the sanitary napkin 20 according to this embodiment has the following advantages.

(1) The topsheet is prevented from clinging to the wearer's skin and causing the wearer discomfort. Since the topsheet under tension is kept in a close contact with the absorbent member, it is prevented from lifting and sticking to the skin for a long time to cause wearing discomfort. When a wearer removes the napkin, which is the time when she may feel most the napkin clinging to her, the topsheet separates from the skin easily, and the wearer can change napkins in a comfortable way.

(2) The topsheet is always in intimate contact with the absorbent member without making a gap therebetween. Therefore, liquid discharged on the topsheet is allowed to migrate to the absorbent member smoothly, hardly remaining in the topsheet. As a result, the napkin is less likely to cause overhydration or a rash, and the liquid is prevented or suppressed from showing its unpleasant color and giving a dirty impression.

(3) Because the topsheet is in close contact under tension with the absorbent member, the absorbent article hardly bunches up. The topsheet, being always united with the absorbent member without lifting alone, prevents the absorbent member from wrinkling or bunching and gives good fit to a wearer.

In order for the topsheet to absorb menstrual blood swiftly and to pass the liquid to the absorbent member smoothly, it is preferred that the portion of the bulky sheet 10 other than the portion forming the leakproof groove 50 be not bonded or be partially bonded to the absorbent member 40 with a hot-melt adhesive applied discretely or by embossing in a discrete pattern.

To ensure the advantages stated above, it is preferred that the bulky sheet 10 be fixed in a state 2 to 30% extended in its MD or CD, that the bulky sheet 10 have a tensile stress of 5 to 40 cN, particularly 10 to 30 cN, at 5% extension in the extending direction, and that the absorbent member have a bending moment of 1 to 30 gf·cm, particularly 3 to 20 gf·cm, in the same direction as the extending direction of the topsheet.

Where the bulky sheet is fixed as extended in the MD, for example, it is preferred that the MD tensile stress of the bulky sheet be in the above-recited range. The MD (machine direction) of the bulky sheet is the running direction of the bulky sheet on a processing machine, and the CD (cross direction) is the direction at right angles to the MD.

The bending moment of the absorbent member is measured in the same direction as the topsheet's extending direction. Where the bulky sheet is fixed as extended in its MD, for example, tensile stress is imposed to the underlying absorbent member in the MD of the bulky sheet. Therefore, the bending moment of the absorbent member is measured in that direction. The bulky sheet 10 is preferably disposed with its MD agreeing with the longitudinal direction or the width direction of the absorbent article.

The tensile stress of the bulky sheet 10 at 5% extension in the MD or CD is measured as follows. The same tensile/compression tester as used in the measurement of extension recovery is used in the tensile mode. A specimen 10 mm wide and 100 mm long is cut out of a sample bulky sheet, with the width perpendicular to the measuring direction, and the length parallel to the measuring direction. The specimen is set between chucks at an initial chuck distance of 50 mm. The chuck attached to the load cell is pulled at a speed of 300 mm/min. The load cell reading at the time when the chuck distance reaches 52.5 cm is taken as a tensile strength at 5% extension. Measurements were made three times to obtain an average.

Where the bulky sheet 10 is fixed at an extension exceeding 30%, the topsheet will exhibit so high contraction stress that the contact with the absorbent member tends to be insufficient for smooth pass of liquid to the absorbent member. Where the extension in fixing is less than 2%, the tensile stress of the topsheet would be insufficient for effectively preventing the absorbent member from wrinkling or bunching.

With the absorbent member having a bending moment of 30 gf·cm or less, the sanitary napkin assures a soft feel while worn. With a bending moment of 1 gf·cm or more, the absorbent member does not greatly bunch up while used. This is especially effective in maintaining the intimate contact with the topsheet and smoothly receiving the liquid.

Where the bulky sheet 10 is fixed in a state extended in both the longitudinal direction and the width direction of an absorbent article, it suffices that the percent extension of the bulky sheet and the bending moment of the absorbent member fall in the above-recited respective ranges in one of the longitudinal direction and the width direction. And yet it is preferred for obtaining more appropriate contractibility of the topsheet and improved contact between the topsheet and the absorbent member that the percent extension and the bending moment be in the above ranges in both the longitudinal direction and the width direction.

It is desirable that the absorbent member 40 be substantially free of curving so as to have increased contact with the topsheet. The phrase "substantially free of curving" as used herein means that the length of an absorbent member under stress measured in the direction parallel to the stress direction is equal to the length of the absorbent member measured in the same direction under no stress.

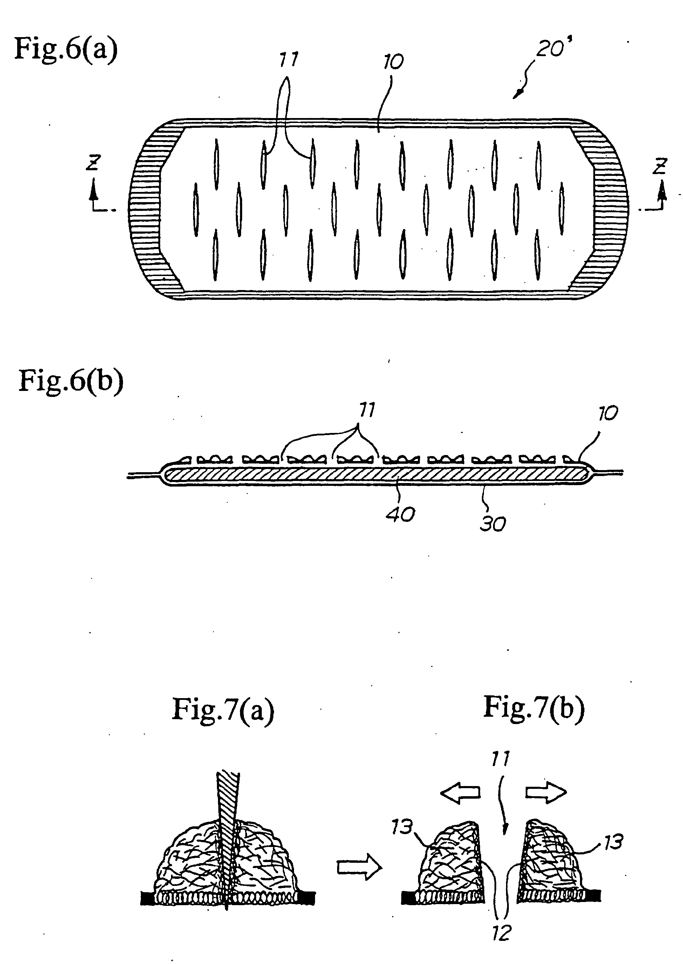

Fig. 6(a) and Fig. 6(b) show another preferred embodiment of the present invention, in which the bulky sheet 10 having the above-described structure has a large number of slits piercing through the first layer 1 and the second layer 2. The slitted bulky sheet 10 is fixed as extended to widen the slits to form openings 11.

The openings 11 open to both the wearer's side and the absorbent member's side are hardly clogged and bring about improved absorbing properties for viscous liquids.

Where the topsheet has expanded slits, i.e., the openings 11 open to the wearer's side, particularly both the wearer's side and the absorbent member's side, liquid permeability from the topsheet to the absorbent member is improved, and the contact area with the skin is reduced to further prevent clinging to the skin thereby giving a wearer comfort free from the unpleasant feeling of the topsheet clinging to her. In the case of the openings piercing through the bulky sheet, in particular, since the inner wall of the openings contains the fiber exhibiting elastomeric behavior, it has freedom of movement to the wearer's movement or pressure. As a result, the openings are kept open to stably produce the above-described effects.

Where the first layer 1 and the second layer 2 are cut through while applying heat to gather the nearby fibers as illustrated in Fig. 7(a) and Fig. 7(b), there will be formed a density gradation from the inner wall 12 of the cut and widened opening 11 to the part 13 farther from the inner wall 12. The density gradation develops an increased force of attracting liquid to give a wearer a dry feel.

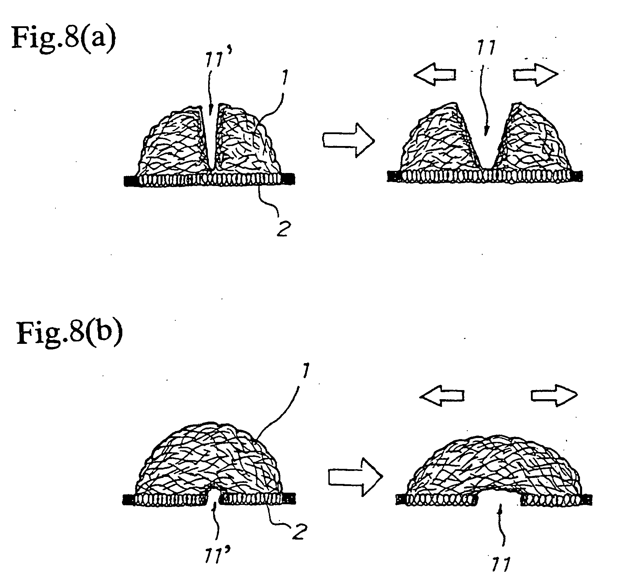

Instead of making slits piercing through the first layer 1 and the second layer 2 as in the sanitary napkin 20' of Fig. 6(a), slits 11' may be made through only one of the first and second layers to form openings 11 when expanded which are open to either the wearer's side or the absorbent member's side as illustrated in Fig. 8(a) and Fig. 8(b). Fig. 8(a) shows slits as cut (11') and as expanded (11) which are open to the wearer's side and produce the same effects as obtained with the slits open to both sides. Fig. 8(b) shows slits as cut (11') and as expanded (11) which are open to the absorbent member's side. The slits of Fig. 8(b) are effective to prevent once absorbed liquid from being squeezed out to the topsheet (a so-called wet-back phenomenon).

In the sanitary napkin 20' of Fig. 6(a), the slits extending in the CD of the bulky sheet 10 (the width direction of the napkin 20') are made in a zigzag pattern, which are to be expanded in the MD (the longitudinal direction of the napkin 20') to make openings 11. It is possible that the slits extending in the MD of the bulky sheet 10 (the longitudinal direction of the napkin 20') are made and expanded in the CD (the width direction of the napkin 20') to form openings.

Materials of the absorbent member and the backsheet are not particularly limited, and any materials that have hitherto been employed in producing absorbent articles, such as sanitary napkins and disposable diapers, can be used. For example, a fiber aggregate, which can contain a superabsorbent polymer, wrapped in a cover sheet of liquid permeable paper or nonwoven can be used as an absorbent member. The fiber aggregate for making the absorbent member includes nonwoven fabric and a fiber web. The superabsorbent polymer is incorporated into the fiber interstices of the fiber aggregate or between layers making up a multilayer fiber aggregate. A diffusing sheet, etc. may be disposed between the topsheet and the absorbent member. The leakproof sheet includes water-repellent nonwoven or paper.

A hydrophilicity difference may be provided between the first layer disposed on the wearer's side and the second layer disposed on the absorbent member's side. Such a hydrophilicity difference is effective in preventing the topsheet from clinging to the wearer's skin and passing liquid from the topsheet to the absorbent member smoothly. A hydrophilicity difference can be made by, for example, selecting a hydrophilizing oil for the fiber constituting the second layer so as to have higher hydrophilicity than a hydrophilizing oil for the fiber constituting the first layer. According to this method, the second layer has higher hydrophilicity than the first layer to attract liquid smoothly. Other methods for imparting higher hydrophilicity to the second layer include making a difference in the coating amount of a hydrophilizing oil between the two layers, incorporating water-absorbing fiber (e.g., cotton) into the second layer, and the like.

The present invention is not limited to the embodiments described *supra.* For example, the fiber aggregates as the first layer 1 and the second layer 2 may be replaced with other materials that are substantially permeable to air. Useful other materials include air permeable films, perforated films, nets, composites of these materials, and composites of these materials and a fiber aggregate.

The second layer 2 may be formed of a fiber aggregate containing elastomer fiber, an elastomer film or an elastomer net! Where such an elastic material is used as a second layer-forming material, the bulky sheet is prepared by, for example, partially joining the second layer-forming material in its extended state with the first layer-forming material by making joints in a prescribed pattern and then releasing the second layer from the extended state. Elastomeric materials which can be used in such an elastic second layer-forming material include natural rubber, isoprene rubber, butadiene rubber, 1,2-polybutadiene, styrene-butadiene rubber, chloroprene rubber, nitrile rubber, butyl rubber, ethylene-propylene rubber, urethane rubber, and various rubbers known as thermoplastic elastomers, such as those comprising styrene, ester, olefin or amide hard segments, and ethylene-α-olefin copolymers obtained by using a metallocene catalyst.

The topsheet used in the present invention is not limited to the bulky sheet having a double layer structure and includes multilayer bulky sheets having three or more layers. For example, for the purpose of enhancing the adhesion to the absorbent member, a third layer may be provided on the second layer on the side opposite to the first layer. The third layer can be of the same fiber aggregate as the first layer-forming material. For the improvement in adhesion to the absorbent member, the material making the third layer is preferably selected from those suitable to adhesion with a hot-melt adhesive or heat-sealing, such as spunbonded nonwovens composed of polyolefin fibers or conjugate fibers containing a polyolefin component.

The absorbent articles according to the present invention include not only sanitary napkins but disposable diapers, incontinence pads, and panty liners.

The present invention will now be illustrated in greater detail with reference to Examples. The following Examples are presented as being exemplary of the present invention and should not be considered as limiting.

EXAMPLE 1

(1) Preparation of first layer-forming material

Heat bondable core-sheath conjugate fiber consisting of a polyethylene terephthalate core and a polyethylene sheath at a core/sheath weight ratio of 5/5 and having a fineness of 2.2 dtex and a fiber length of 51 mm (NBF-SH, available from Daiwabo Co., Ltd.) was carded into a web and heat treated at 120°C to prepare a nonwoven fabric having a basis weight of 15 $g/m^2$.

(2) Preparation of second layer-forming material

Self-crimping fiber which was heat shrinkable core-sheath conjugate fiber consisting of an ethylene-propylene random copolymer (EP) core and a polypropylene (PP) sheath and having a fineness of 2.2 dtex and a shrinkage starting temperature T, of 90° (available from Daiwabo Co., Ltd.) was carded into a web. The resulting nonwoven fabric had a basis weight of 35 $g/m^2$.

(3) Preparation of bulky sheet

The nonwoven fabrics prepared in (1) and (2) above were superposed on each other and passed through a heat embossing machine composed of an engraved roll and a smooth roll to join them. The engraved roll temperature was set at 155°C. The engraved roll had projections each having a circular shape with an emboss area of 0.047 $cm^2$ arrayed at a pitch of 7 mm (L1 and L2) in both the MD and the CD and a pitch of 5 mm (L3) in the direction forming 45° with the MD and the CD to totally make the pattern of Fig. 3. The total emboss area ratio was 7.2%.

The four sides of the resulting laminate of the two nonwoven fabrics were fixed on the pins of a pin tenter adjusted to the area to which the laminate was to shrink so that the laminate might not overshrink more than designed. The laminate as caught on the pin tenter was heat treated in a dryer set at 130°C for 1 to 3 minutes to obtain a bulky sheet having a basis weight of 100 $g/m^2$.

(4) Preparation of sanitary napkin (absorbent article)

The resulting bulky sheet was cut into a rectangle 125 mm wide in the CD and 230 mm long in the MD. The cut sheet was 10% extended in the MD and placed as extended on an absorbent member having a pulp content of 210 $g/m^2$ and an absorbent polymer content of 40 $g/m^2$, which was the same as used in a commercially available sanitary napkin, Laurier Sarasara Cushion Slim (with no wings) supplied from Kao Corp. (hereinafter referred to its sale name "Laurier UN-f-11") with the first layer on the wearer's side and the MD of the bulky sheet in agreement with the longitudinal direction of the absorbent member. The bulky sheet in the extended state and the absorbent member were united by embossing. The embossing was carried out in a stamping press having a sandglass-shaped loop embossing pattern (the one used in the manufacture of Laurier UN-f-11) to make a leakproof groove 50. The thus united bulky sheet and absorbent member were assembled with other members, such as a backsheet and a leakproof sheet, to obtain a sanitary napkin having the configuration shown in Fig. 5(a) and Fig. 5(b).

The backsheet and leakproof sheet used in Example 1 were the same as those used in Laurier UN-f-11. The backsheet was a polyethylene film having a basis weight of 25 $g/m^2$. The leakproof sheet was a polyethylene-laminated water-repellent paper having a basis weight of 23 $g/m^2$. The backsheet was 75 mm wide and 210 mm long, and the leakproof sheet was 105 mm wide and 210 mm long. The backsheet was bonded to the leakproof sheet provided on the back side of the absorbent member via a hot-melt adhesive 70 (see Fig. 5(b)) which was applied in two strips 20 mm wide and 130 mm long each at a spread of 30 g/m$^2$.

The materials of other members and the structure of the sanitary napkin were the same as those of Laurier UN-f-11 except the following. Although Laurier UN-f-11 has a side leakproof groove formed by emobssing on each outer side of the leakproof groove 50, these side leakproof grooves were not formed on the sanitary napkin of Example 1.

The topsheet in the portion surrounded by the loop of the leakproof groove 50 was flat and substantially free of curving. No curving in the MD was observed with the absorbent member and the whole product, either.

EXAMPLE 2

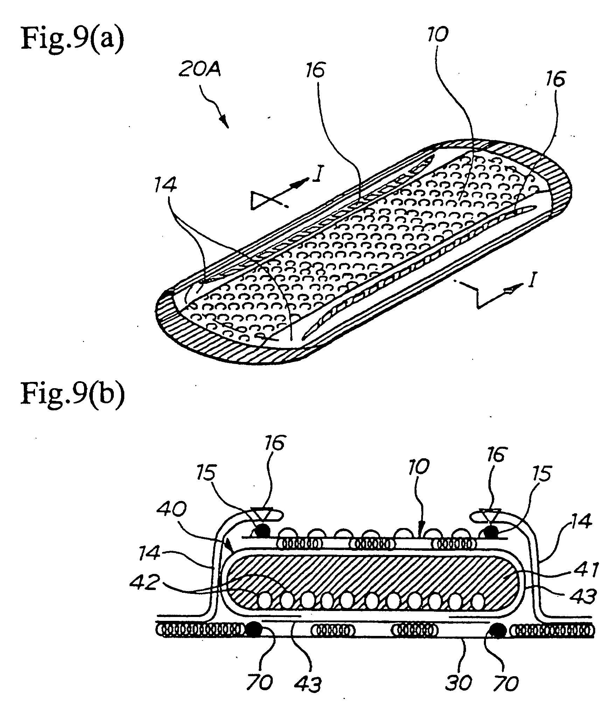

The bulky sheet prepared in Example 1 was cut into a rectangle 80 mm wide in the CD and 230 mm long in the MD. Separately, two 60 mm wide strips of water-repellent spunbonded nonwoven fabric having a basis weight of 18 g/m$^2$ made of core-sheath conjugate fiber consisting of a polyethylene terephthalate core and a polyethylene sheath were prepared. Each of the spunbonded nonwoven fabric strips was folded into two along its longitudinal direction to prepare a pair of 30 mm wide strips 14 (folded) (see Fig. 9(a) and Fig. 9(b)).

The cut bulky sheet 10 was 10% extended in the MD. The mountain-folded side of the folded nonwoven fabric strip 14 was bonded with a hot-melt adhesive 15 to each longitudinal side of the extended bulky sheet 10 with a 10 mm overlap. The resulting composite sheet had a 60 mm wide portion of the bulky sheet 10 and a 30 mm wide two-folded spunbond nonwoven fabric 14 on both sides.of the bulky sheet.

The composite sheet was placed on the same absorbent member as used in Laurier UN-f-11 (pulp content: 210 g/m$^2$; absorbent polymer content: 40 g/m$^2$), and the bulky sheet 10 in its extended state was joined with the absorbent member 40 by embossing the overlap of the bulky sheet 10 and the two-folded spunbond nonwoven fabric 14 to form grooves 16. The peripheral portion of the composite sheet extending over the periphery of the absorbent member 40 was bonded to a backsheet 30 (the same as used in Laurier UN-f-11; a polyethylene film having a basis weight of 25g/m$^2$) disposed under the absorbent member 40 to make a sanitary napkin shown in Fig. 9(a) and Fig. 9(b) . The resulting sanitary napkin was not substantially curved.

As shown in Fig. 9(b), the absorbent member used here had an absorbent core comprising pulp fiber 41 and an absorbent polymer 42 wrapped in absorbent paper 43. A hot-melt adhesive was applied to the upper and lower sides of the absorbent member 40 in a spiral pattern in parts, and the bulky sheet 10 and the backsheet 30 were adhered to the respective sides via the adhesive.

The configuration of the sanitary napkin of Example 2, in which the bulky sheet 10 with a reduced width was disposed only on the widthwise middle of the napkin, is economically advantageous from the standpoint of material cost. Use of the bulky sheet with a reduced width is also advantageous in that the shrinkage stress imposed to the whole topsheet is decreased. It follows that the need to increase the rigidity (bending rigidity) of the absorbent member can be reduced.

EXAMPLE 3

A sanitary napkin was prepared in the same manner as in Example 2, except that the composite sheet was fixed with its bulky sheet portion 15% extended in the MD.

EXAMPLE 4

A sanitary napkin was prepared in the same manner as in Example 2, except for using the absorbent member used in another product of Kao Corp., Laurier Yawaraka Mesh Superslim Guard (sale name: Laurier PW-c).

EXAMPLE 5

The bulky sheet prepared in Example 1 was cut to make a large number of slits parallel to the CD and piercing both the first and second layers in the following regular pattern.

Slit length in the CD: 3 mm

Slit pitch in the MD: 5 mm

Number of slits in a row extending in the MD: 18

MD length of the row of slits: 85 mm

Number of rows (each extending in the MD) of slits counted in the CD: 6

Distance between adjacent rows of slits in the CD: 3 mm

Difference in position in the MD between slits in adjacent rows (each extending in the MD) of slits: $\pm 2.5$ mm

A sanitary napkin was prepared in the same manner as in Example 3, except for using the slitted bulky sheet. The topsheet of the resulting sanitary napkin had a large number of openings (expanded slits) in the middle portion in both the longitudinal and width directions of the product.

EXAMPLE 6

The bulky sheet prepared in Example 1 was cut to make a large number of slits parallel to the MD and piercing both the first and second layers in the following regular pattern.
Slit length in the MD: 3 mm
Distance between adjacent slits in the MD: 3 mm
Slit pitch in the MD: 6 mmNumber of slits in a row extending in the MD: 14
MD length of the row of slits: 81 mm
Number of rows (each extending in the MD) of slits counted in the CD: 10
Distance between adjacent slits in the CD (=slit pitch in the CD): 3 mm
Difference in position in the MD between slits in adjacent rows (each extending in the NM) of slits: $\pm 3$ mm
A sanitary napkin was prepared in the same manner as in Example 2, except for using the slitted bulky sheet and fixing the bulky sheet as extended 10% in the width direction of the sanitary napkin to the absorbent member. The topsheet of the resulting sanitary napkin had a large number of openings (expanded slits) in the middle portion in both the longitudinal and width directions of the product.

COMPARATIVE EXAMPLE 1

A sanitary napkin was prepared in the same manner as in Example 2, except that the bulky sheet was fixed to the absorbent member without being extended.

COMPARATIVE EXAMPLE 2

A sanitary napkin was prepared in the same manner as for another product of Kao Corp., Laurier Yawaraka Mesh Sarasara Cushion. Slim (with wings) (sale name: Laurier UN-f), except that the topsheet was fixed in a state 10% extended in the MD (the same as the longitudinal direction of the product) to the absorbent member. The topsheet used here was a perforated nonwoven fabric made of core/sheath conjugate fiber having a polyethylene terephthalate core and a polyethylene sheath. It had 10 to 15 perforations having a diameter of about 0.8 to 1 mm per square centimeter.

Evaluation:

1) The bulky sheets used as a topsheet of the sanitary napkins of Examples and Comparative Examples were measured for the height of the protrusions and the tensile stress at 5%, 10%, 15% and 20% extension in the MD and the CD in accordance with the methods described *supra*. The absorbent members used in these sanitary napkins were measured for the bending moment in the same direction as the extending direction of the bulky sheets in accordance with JIS P8125 (Taber Stiffness Tester Method, see below). The results obtained are shown in Table 1 below.

Method of measuring bending moment:

Measurement was made using a taber stiffness tester supplied by Kumagai Riki Kogyo Co., Ltd. A 38 mm wide and 70 mm long specimen was cut out of a sample absorbent member (the width of the specimen agreeing with the width of the absorbnet member). A force was applied to one end of the specimen to induce a 15° bend at a speed of $180° \pm 40°$/min, and the bending moment was measured. The mean value of the bending moments in opposing directions was recorded. Three measurements were made per sample to obtain an average.
In Example 6, since the bulky sheet was fixed to the absorbent member as extended in its CD, the bending moment shown in Table 1 is the one measured by bending the absorbent member in its width direction which corresponded to the CD of the bulky sheet. The used specimen was prepared by cutting out by 70 mm in the width and 38 mm in the length of the absorbent member.

2) Curving of absorbent article

The sanitary napkin was inspected for curving in the longitudinal direction. The results are shown in Table 1. Products free from curving in the longitudinal direction are those of which the topsheet is not substantially curved in the longitudinal direction of the products. The degree of curving was expressed in curvature radius (R) measured as follows.

A curvature radius R is calculated from a linear distance A of two longitudinal ends of a curved absorbent member and a depth B of the curve (the depth from the bisecting point of the line segment A) by making use of the Pythagorean theorem according to equation.

$$\text{Curvature radius } R = [(1/4)A^2 + B^2]/2B$$

3) Contact between topsheet and absorbent member

A sanitary napkin was cut along a line parallel to the extending direction of the topsheet, and the cut area was observed. Those products having a gap exceeding 1 mm between the topsheet and the absorbent member were regarded as having no contact. Those having a gap of 1 mm or smaller were regarded as having contact, taking the back side unevenness of the topsheet into consideration. The degree of contact in the latter samples was rated A (close contact) or B (contact) according to the following standard. The samples having no contact were rated C. The results of rating are shown in Table 1.

    A: The topsheet is in close contact with the absorbent member with a gap of 1 mm or smaller.
    B. The topsheet is in contact with the absorbent member with a gap of 1 mm or smaller, but the topsheet is apt to lift.
    C: There is a gap exceeding 1 mm between the topsheet and the absorbent member.

4) Amount of remaining liquid

The amount of liquid remaining in the topsheet was measured as follows to evaluate liquid migration from the topsheet to the absorbent member. The results obtained are shown in Table 1.

A sanitary garment (Laurier Shorts, standard shorts, L size, available from Kao Corp.) having a sanitary napkin stuck to the crotch portion thereof was put on a movable female body model supplied by Takaken K.K. The topsheet used in the sanitary napkin, which had a cut size of 75 mm by 205 mm, had previously been weighed. After the model was operated to take a walking movement of 100 steps/mi n for 1 minute, 2 g of defibrinated horse blood (available from Nippon Biotest Lab.) was poured into the napkin at a rate of 0.13 g/sec, and the model was operated to take the same walking movement for an additional 29 minute period. After the test, the topsheet was weighed again. The weight gain of the topsheet is the amount of the liquid remaining in the topsheet. The test was conducted three times to obtain an average value.

5) Liquid spread area

How smoothly the topsheet passes liquid to the absorbent member, which owes to the intimate contact between the topsheet and the absorbent member, was evaluated in terms of area that a liquid spreads. The area a liquid spread was measured as follows. The results obtained are shown in Table 1.

A sanitary napkin was stuck to the same sanitary garment used above and was put on the same female body model as used above, which was set to take a walking movement at a speed of 100 steps/min. After the model was operated to take a walking movement of 100 steps/min for I minute, 3 g of defibrinated horse blood (available from Nippon Biotest Lab.) was poured into the napkin at a rate of 0.13 g/sec, and the model was operated to take the same walking movement for an additional 29 minute period. After the test, the outline of the area of the topsheet where the liquid had spread was traced on a transparent sheet. The outlined area of the transparent sheet was measured.

TABLE 1

| | | Example | | | | | | Comp. Example | |
|---|---|---|---|---|---|---|---|---|---|
| | | 1 | 2 | 3 | 4 | 5 | 6 | 1 | 2 |
| Topsheet (Bulky Sheet): | | | | | | | | | |
| 1st Fiber Layer | Basis Weight (g/m²) | 15 | 15 | 15 | 15 | 15 | 15 | 15 | 25 |
| | Slit | none | none | none | none | made | made | none | none |
| 2nd Fiber Layer | Basis Weight (g/m²) | 35 | 35 | 35 | 35 | 35 | 35 | 35 | - |
| | Slit | none | none | none | none | made | made | none | - |
| Protrusion Height (mm) | | 2.60 | 2.60 | 2.60 | 2.60 | 2.55 | 2.53 | 2.60 | - |
| MD Tensile Stress (cN) | 5% Extension | 23 | 23 | 23 | 23 | | | 23 | 60 |
| | 10% Extension | 47 | 47 | 47 | 47 | | | 47 | 187 |
| | 15% Extension | 151 | 151 | 151 | 151 | | | 151 | 301 |
| | 20% Extension | 325 | 325 | 325 | 325 | | | 325 | 341 |
| CD Tensile Stress (cN) | 5% Extension | 25 | 25 | 25 | 25 | | | 25 | 5 |
| | 10% Extension | 54 | 54 | 54 | 54 | | | 54 | 13 |
| | 15% Extension | 120 | 120 | 120 | 120 | | | 120 | 46 |
| | 20% Extension | 210 | 210 | 210 | 210 | | | 210 | 88 |
| Absorbent Article (Sanitary Napkin): | | | | | | | | | |
| Extension of Topsheet (%) | MD | 10 | 10 | 15 | 10 | 15 | - | - | 10 |
| | CD | - | - | - | - | - | 10 | - | - |
| Bending Moment of Absorbent Member (gf·cm) | | 8.6 | 8.6 | 8.6 | 3.5 | 8.6 | 5.8 (CD) | 8.6 | 8.6 |
| Curvature Radius (mm) in Longitudinal Direction | | no curving | no curving | 230 | 220 | no curving | no curving | no curving | 215 |
| Contact between Topsheet and Absorbent Member | | A (close contact) | A (close contact) | A (close contact) | A (close contact) | A (close contact) | A (close contact) | B (contact) | C (no contact) |
| Amount of Remaining Liquid (mg) | | 75 | 78 | 70 | 86 | 65 | 68 | 100 | 48 |
| Liquid Spread Area (cm²) | | 7 | 7 | 6 | 7.5 | 6 | 6 | 10 | 28 |

EP 1 346 712 B1

The sanitary napkins of Examples 1 to 6 had smaller amounts of liquid remaining in the topsheet than the napkin of Comparative Example I having the bulky sheet fixed in its non-extended state, which proves sufficient contact between the topsheet and the absorbent member and smooth pass of the liquid to the absorbent member. Further, the napkins of Examples had smaller liquid spread areas than the napkin of Comparative Example 1 having the bulky sheet fixed in the non-extended state and the napkin of Comparative Example 2 having the topsheet as used in a commercially available sanitary napkin in its extended state, which indicates that the topsheets in the napkins of Examples were in sufficient contact with the absorbent members to allow the liquid to migrate to the absorbent members more smoothly.

The present invention provides an absorbent article the topsheet of which hardly clings to the skin and which allows liquid to smoothly migrate from the topsheet to the absorbent member thereby to prevent overhydration, a rash, and a dirty impression due to the color of the liquid, and hardly bunches up.

## Claims

1. An absorbent article having a substantially oblong shape and comprising a liquid permeable topsheet, a liquid impermeable backsheet, and a liquid retentive absorbent member interposed between the topsheet and the backsheet, wherein said topsheet is a bulky sheet which can exhibit elastomeric behavior as a whole and comprises a first layer disposed on the side of a wearer and a second layer disposed on the side of the absorbent member, said first layer and said second layer being partially bonded together at joints in a prescribed pattern, said first layer having protrusions on the side of a wearer in portions other than the joints, said second layer being made of a material exhibiting elastomeric behavior, and said topsheet is fixed in an extended state to the upper side of the absorbent member in at least one of the longitudinal direction and the width direction of the absorbent article.

2. The absorbent article according to claim 1, wherein said topsheet has cuts in at least one of the first layer and the second layer, and the cuts are adapted to be expanded by the extension of said topsheet to make holes open to at least one of the side of a wearer and the side of the absorbent member.

3. The absorbent article according to claim 1, wherein said topsheet is fixed in a state 2 to 30% extended in the machine direction or the cross direction thereof and has a tensile stress of 5 to 40 cN at 5% extension in the extending direction, and said absorbent member has a bending moment of 1 to 30 gf·cm in the same direction as the extending direction of said topsheet.

4. The absorbent article according to claim 1, wherein, before use, said absorbent member is substantially free of curving.

5. The absorbent article according to claim 1, wherein the second layer of said topsheet is a fiber aggregate containing a fiber which comprises a thermoplastic polymer and which has heat shrinkability to exhibit elastomeric behavior, and the first layer of said topsheet is a fiber aggregate containing a fiber which comprises a thermoplastic polymer and which has substantially no heat shrinkability or does not shrink at the shrinkage starting temperature of said fiber having heat shrinkability.

6. The absorbent article according to claim 5, wherein the second layer is a fiber aggregate containing a self-crimping fiber.

## Patentansprüche

1. Absorbierender Artikel mit einer im wesentlichen länglichen Form, der eine flüssigkeitsdurchlässige oberste Schicht, eine flüssigkeitsundurchlässige Rückenschicht und ein zwischen der obersten Schicht und der Rückenschicht angeordnetes flüssigkeitszurückhaltendes absorbierendes Element aufweist, wobei die oberste Schicht eine bauschige Schicht ist, die als Ganzes ein elastomeres Verhalten zeigen kann und die aufweist: eine auf der Seite eines Trägers angeordnete erste Schicht und eine auf der Seite des absorbierenden Elements angeordnete zweite Schicht, wobei die erste Schicht und die zweite Schicht an Verbindungen in einem vorgeschriebenen Muster teilweise miteinander verbunden sind, wobei die erste Schicht auf der Trägerseite in anderen Teilen als den Verbindungen Vorsprünge hat, die zweite Schicht aus einem Material gefertigt ist, das ein elastomeres Verhalten zeigt, und die oberste Schicht in einem in der Längsrichtung und/oder der Breitenrichtung des absorbierenden Artikels gedehnten Zustand an der Oberseite des absorbierenden Elements befestigt ist.

**2.** Absorbierender Artikel nach Anspruch 1, wobei die oberste Schicht Ausschnitte in der ersten Schicht und/oder der zweiten Schicht hat, und die Ausschnitte geeignet sind, durch die Dehnung der obersten Schicht gedehnt zu werden, um Löcher zu bilden, die auf der Trägerseite und/oder der Seite des absorbierenden Elements offen sind.

**3.** Absorbierender Artikel nach Anspruch 1, wobei die oberste Schicht in einem Zustand mit 2 bis 30% Dehnung in der Maschinenrichtung oder deren Querrichtung befestigt wird und sie bei 5% Dehnung in der Dehnungsrichtung eine Zugspannung von 5 bis 40 cN hat, und das absorbierende Element in der gleichen Richtung wie der Dehnungsrichtung der obersten Schicht ein Biegemoment von 1 bis 30gf·cm hat.

**4.** Absorbierender Artikel nach Anspruch 1, wobei das absorbierende Element vor der Verwendung im wesentlichen krümmungsfrei ist.

**5.** Absorbierender Artikel nach Anspruch 1, wobei die zweite Schicht der obersten Schicht eine Faseranhäufung ist, die eine Faser enthält, welche ein thermoplastisches Polymer aufweist und die ein Wärmeschrumpfungsvermögen hat, um ein elastomeres Verhalten zu zeigen, und wobei die erste Schicht der obersten Schicht eine Faseranhäufung ist, die eine Faser enthält, welche ein thermoplastisches Polymer aufweist und die im wesentlichen kein Wärmeschrumpfungsvermögen hat oder bei der Schrumpfungsanfangstemperatur der Faser mit dem Wärmeschrumpfungsvermögen nicht schrumpft.

**6.** Absorbierender Artikel nach Anspruch 5, wobei die zweite Schicht eine Faseranhäufung ist, die eine selbstkräuselnde Faser enthält.

**Revendications**

**1.** Article absorbant ayant une forme sensiblement oblongue, et comportant une feuille supérieure perméable à un liquide, une feuille arrière imperméable à un liquide, et un élément absorbant rétenteur de liquide interposé entre la feuille supérieure et la feuille arrière, dans lequel ladite feuille supérieure est une feuille volumineuse qui peut présenter un comportement élastomérique dans son ensemble, et comporte une première couche disposée du côté d'un utilisateur et une seconde couche disposée du côté de l'élément absorbant, ladite première couche et ladite seconde couche étant partiellement liées ensemble au niveau de joints selon un motif prescrit, ladite première couche ayant des saillies du côté de l'utilisateur dans des parties autres que les joints, ladite seconde couche étant constituée d'un matériau présentant un comportement élastomérique, et ladite feuille supérieure est fixée dans un état étendu sur le côté supérieur de l'élément absorbant dans au moins une direction parmi la direction longitudinale et la direction latérale de l'article absorbant.

**2.** Article absorbant selon la revendication 1, dans lequel ladite feuille supérieure a des entailles dans au moins une couche parmi la première couche et la seconde couche, et les entailles sont adaptées pour être agrandies par l'agrandissement de ladite feuille supérieure pour réaliser des trous ouverts vers au moins un côté parmi le côté de l'utilisateur et le côté de l'élément absorbant.

**3.** Article absorbant selon la revendication 1, dans lequel ladite feuille supérieure est fixée dans un état étendu de 2 à 30 % dans la direction de la machine ou dans la direction transversale à la direction de la machine, et a une résistance à la traction de 5 à 40 cN à 5 % d'extension dans la direction d'extension, et ledit élément absorbant a un moment de flexion de 1 à 30 gf · cm dans la même direction que la direction d'extension de ladite feuille supérieure.

**4.** Article absorbant selon la revendication 1, dans lequel, avant utilisation, ledit élément absorbant est sensiblement sans courbure.

**5.** Article absorbant selon la revendication 1, dans lequel la seconde couche de ladite feuille supérieure est un agrégat de fibres contenant une fibre qui comporte un polymère thermoplastique et qui a une capacité à être thermorétractable pour présenter un comportement élastomérique, et la première couche de ladite feuille supérieure est un agrégat de fibres contenant une fibre qui comporte un polymère thermoplastique et qui n'a sensiblement pas d'aptitude à être thermorétractable ou n'est pas thermorétractable à la température de démarrage de rétraction de ladite fibre ayant une aptitude à être thermorétractable.

**6.** Article absorbant selon la revendication 5, dans lequel la seconde couche est un agrégat de fibres contenant une fibre se gaufrant automatiquement.

## Fig.1

## Fig.2

## Fig.3

## Fig.4(a)

## Fig.4(b)

## Fig.4(c)

Fig.5(a)

Fig.5(b)

16

## Fig.6(a)

## Fig.6(b)

## Fig.7(a)        Fig.7(b)

Fig.8(a)

Fig.8(b)

Fig.9(a)

Fig.9(b)